# EUROPEAN PATENT APPLICATION

(11) **EP 3 354 215 A1**
(43) Date of publication of application: **01.08.2018**
(21) Application number: 18153331.6
(22) Date of filing: 24.01.2018
(51) Int. Cl.: A61B 17/70

(54) **INTERSPINOUS STABILIZER**

(30) Priority: 24.01.2017 TW 106103150
(71) Applicant: Love U Co., Ltd., 807 Kaohsiung City (TW); BAUI Biotech Co., Ltd., New Taipei City (TW)
(72) Inventor: HWANG, SHIUH-LIN, 833 Kaohsiung City (TW); LIN, CHIEN-YU, New Taipei City (TW)
(74) Representative: CMS Cameron McKenna Nabarro Olswang LLP

(57) **Abstract**

An interspinous stabilizer (2), comprising: an interspinous spacer (1), comprising a central support (100), having a first direction (13) and a second direction (14) opposite to each other, and an upper side (11) and a lower side (12) perpendicular to the first direction and the second direction; an upper side wing (110) is on the first direction and extending from the upper side of the central support; a lower side wing (111) is on the first direction and extending from the lower side of the central support; an upper protrusion (120) is on the second direction and extending from the upper side of the central support; a lower protrusion (121) is on the second direction and extending from the lower side of the central support; at least one perforation (140), the perforation traversing through the first direction to the second direction of the central support.

## Description

### FIELD

The present disclosure relates to medical devices. Specifically, the interspinous spacer and the interspinous stabilizer used for treating neural compression caused by degenerative spinal disease or spinal stenosis.

### BACKGROUND

The human spine is composed of many vertebrae and intervertebral discs, and the aging or degenerating of any one of the intervertebral discs would lead to spinal stenosis, thus the nearby spinal nerves may be compressed. The compression of spinal nerves may lead to impaired movement or pain to the patient.

Regarding the above discomforts and disorder caused by spinal stenosis, a common surgical approach is to implant an interspinous spacer or an interspinous stabilizer between 2 spinous processes on the dorsal side of the spine. However, commercially available interspinous spacers or interspinous stabilizers are mostly made of rigid materials, and they do not fit the vertebrae structure. Moreover, interspinous spacers or interspinous stabilizers may cause tissue damages around the spinous processes.

Wallis® from Abbott Spine, Coflex® from Paradigm Spine and X-Stop®
from Medtronic are interspinous stabilizers made from metal material. U.S. Patent No. 7,955,392 disclosed an interspinous spacer, and U.S. Patent No. 8,968,365 disclosed a rigid interspinous stabilizer with elasticity. However, all of the above interspinous stabilizers or interspinous spacers are made of stainless steel, titanium alloy or polyetheretherketone (PEEK), thus they are unable to fit the structure of the spinous processes. Therefore, they may induce concentrated stress on the spinous processes, leading to fracture of the spinous processes; the above rigid interspinous stabilizers may be dislocated due to the flexion, lateral flexion and rotation of the patient's spine.

U.S. Patent No. 8,118,839 disclosed a soft interspinous spacer; DIAM® from
Medtronic and Interspine® from Cousin Biotech are soft interspinous spacers
composed mainly of silicone, therefore they are compressible and elastic interspinous spacers. The soft interspinous spacers also prevent the fracture of interspinous processes due to concentrated stress when using rigid interspinous stabilizers.

Each of the above soft interspinous spacers has double-wing structure, namely, wing-shaped protrusions of identical sizes are present at both directions of the interspinous spacer. The symmetrical double-wing structure provides a better fitting for the interspinous spacer between 2 spinous processes. However, the above symmetrical double-wing structure is easier to be implanted by the surgeon from the rear side of the spinous process. It would be more difficult for the above symmetrical double-wing structure to be implanted from the lateral side of the spinous process, and the fracture of the spinous process may occur if the symmetrical double-wing structure are being implanted from the lateral side of the spinous process.

Also, after being implanted between the spinous processes, DIAM®
interspinous spacer need to be fixed by 2 cables. Namely, 2 cables need to be pulled separately by the surgeon to fix the interspinous spacer onto a precise position between the spinous processes. Consequently, the uneven pulling of 3 cables attributes to each of the parts of the spine being tied by the cables bearing different level of stress, and this greatly increases the possibility of dislocation of the interspinous spacer.

In light of the above, an interspinous stabilizer capable to solve the previous technical problems is needed. The interspinous stabilizer needs to be implanted from the rear side or lateral side of the spinous process, be easier to operate for the surgeon, and have a proper cable fixation mechanism to increase the stability of the spacer and decrease the possibility of dislocation.

### BRIEF DESCRIPTION OF THE DRAWINGS

The present description will be better understood from the following detailed description read in light of the accompanying drawings, where:
FIG. 1 is the appearance of an interspinous spacer in accordance with aspects of the present disclosure.
FIG. 2 is the appearance of an interspinous stabilizer in accordance with aspects of the present disclosure.
FIG. 3 is the appearance of an interspinous stabilizer and its' fabric sheath in accordance with aspects of the present disclosure.
FIG. 4 is the appearance of the band in accordance with aspects of the present disclosure.
FIG. 5 is the perspective view of an interspinous spacer in accordance with aspects of the present disclosure.
FIG. 6 is the side view of an interspinous stabilizer from the first direction in accordance with aspects of the present disclosure.
FIG. 7 is the side view of an interspinous stabilizer and its' fabric sheath from the first direction in accordance with aspects of the present disclosure.
FIG. 8 is the side view of an interspinous stabilizer from the second direction in accordance with aspects of the present disclosure.
FIG. 9 is the side view of an interspinous stabilizer and its' fabric sheath from the second direction in accordance with aspects of the present disclosure.
FIG. 10 is the side view of an interspinous spacer from the first direction in accordance with aspects of the present disclosure.
FIG. 11 is the side view of an interspinous spacer from the second direction in accordance with aspects of the present disclosure.
FIG. 12 is the cross-sectional view of an interspinous spacer from the first direction in accordance with aspects of the present disclosure.
FIG. 13 is the appearance of another interspinous spacer in accordance with aspects of the present disclosure.
FIG. 14 is the appearance of another interspinous stabilizer in accordance with aspects of the present disclosure.
FIG. 15 is the appearance of another interspinous stabilizer and its' fabric sheath in accordance with aspects of the present disclosure.
FIG. 16 is the perspective view of another interspinous spacer in accordance with aspects of the present disclosure.
FIG. 17 is the side view of another interspinous spacer from the first direction in accordance with aspects of the present disclosure.
FIG. 18 is the side view of another interspinous spacer from the second direction in accordance with aspects of the present disclosure.
FIG. 19 is the top view of an interspinous stabilizer implanted between the spinous processes in accordance with aspects of the present disclosure.
FIG. 20 is the top view of another interspinous stabilizer implanted between the spinous processes in accordance with aspects of the present disclosure.
FIG. 21 is the side view of another interspinous stabilizer implanted between the spinous processes in accordance with aspects of the present disclosure.

### DETAILED DESCRIPTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

An embodiment of the present disclosure provides an interspinous stabilizer. The interspinous stabilizer comprises a soft interspinous spacer, and the interspinous spacer having an asymmetrical double-wing structure for easier insertion between the spinous processes. The interspinous stabilizer having only one band, and the band is pulled when the interspinous spacer is in need of being positioned after the insertion. The different parts of the spine thus bear similar levels of stress, and the interspinous stabilizer is tied by a band to decrease the chance of interspinous stabilizer dislocation. "Stress" refers to the force per unit area.

An embodiment of the present disclosure provides an interspinous spacer for use in the interspinous stabilizer, comprising a central support having a first direction and a second direction opposite to each other, an upper side perpendicular to the first direction and the second direction, and a lower side perpendicular to the first direction and the second direction. An upper side wing is on the first direction and extending from the upper side of the central support; a lower side wing is on the first direction and extending from the lower side of the central support. An upper protrusion is on the second direction and extending from the upper side of the central support, wherein a length of the upper protrusion is shorter than a length of the upper side wing. A lower protrusion is on the second direction and extending from the lower side of the central support, wherein a length of the lower protrusion is shorter than a length of the lower side wing; and at least one perforation, the perforation extending through the central support from the first direction to the second direction. The interspinous spacer in accordance with the present disclosure has an asymmetrical double-wing structure, as the upper side wing is longer than the upper protrusion and the lower side wing is longer than the lower protrusion. The asymmetrical double-wing structure makes it easier for a lateral side insertion of the interspinous spacer between 2 vertebrae.

An embodiment of the present disclosure provides another interspinous spacer to be used in the interspinous stabilizer, comprising a central support having a first direction and a second direction opposite to each other, an upper side perpendicular to the first direction and the second direction, and a lower side perpendicular to the first direction and the second direction. An upper side wing is on the second direction and extending from the upper side of the central support; a lower side wing is on the second direction and extending from the lower side of the central support. An upper protrusion is on the first direction and extending from the upper side of the central support, wherein a length of the upper protrusion is shorter than a length of the upper side wing. A lower protrusion is on the first direction and extending from the lower side of the central support, wherein a length of the lower protrusion is shorter than a length of the lower side wing; and at least one perforation, the perforation extending through the central support from the first direction to the second direction. The interspinous spacer in accordance with the present disclosure has an asymmetrical double-wing structure, as the upper side wing is longer than the upper protrusion and the lower side wing is longer than the lower protrusion. The asymmetrical double-wing structure makes it easier for a lateral side insertion of the interspinous spacer between 2 vertebrae.

The central support of the interspinous spacer in accordance with an embodiment of the present disclosure further comprises an upper support and a lower support. The upper support is on the upper side of the central support and is connected to the upper protrusion, and the lower support is on the lower side of the central support and is connected to the lower protrusion. The interspinous spacer in accordance with an embodiment of the present disclosure further comprises an upper perforation and a lower perforation. The upper perforation and the lower perforation extending through the central support, and the upper perforation and the lower perforation is in the upper support.

Each of the upper side wing, the lower side wing, the upper protrusion and the lower protrusion comprise a root portion close to the central support and an end portion away from the central support. Each of the upper side wing, the lower side wing, the upper protrusion and the lower protrusion has a transitional edge. The transitional edges enable the interspinous spacer to be laterally implanted, and the interspinous spacer would be easier to be implanted between 2 vertebrae.

The central support of the interspinous spacer in accordance with an embodiment of the present disclosure further comprises a front side and a rear side, and each of the front side and the rear side is perpendicular to the first direction and the second direction, and each of the front side and rear side is perpendicular to the upper side and the lower side. A horizontal plane is formed from the front side extending to the rear side, wherein the horizontal plane is perpendicular to the upper side and the lower side. The central support further comprises an upper concave surface between the upper side wing and the upper protrusion, and the upper concave surface is on the upper side of the central support, and the upper concave surface slopes down from the front side to the rear side and forms an upper angle; a lower concave surface is between the lower side wing and the lower protrusion, and the lower concave surface is on the lower side of the central support. The upper concave surface is not parallel with the lower concave surface, thus are conformed with the structure of human spine. The interspinous spacer is therefore tightly conformed with the two vertebrae of the patient.

The interspinous stabilizer in accordance with the present disclosure further comprises a band, and the band can pass through the perforation. The band can form a circular structure on the first direction or the second direction of the central support. The interspinous stabilizer further comprises at least one metal hook, the metal hook is placed on at least one end of the band, wherein the metal hook can be guided to pass through the circular structure to form a knot. The band, the metal hook, the circular structure and the knot allows the interspinous stabilizer to be conveniently fixed between the vertebrae. The different parts of the vertebrae being tied by the band receive similar levels of stress because of the single band design, and the interspinous stabilizer may not be dislocated once it was implanted.

The interspinous stabilizer in accordance with the present disclosure further comprises a fabric sheath, and the fabric sheath may wrap all of the interspinous spacer. The fabric sheath of the present disclosure can be a cushion between the interspinous stabilizer and the spine, to prevent the abrasion of the interspinous spacer due to the direct contact between the interspinous spacer and the vertebrae, and effectively reduces the damage from the interspinous spacer to the surrounding tissues of the spinous processes.

The present disclosure provides a method of stabilizing relative position between the human spinous processes, comprising: (i) insert an interspinous stabilizer between two target spinous processes of a spine; (ii) pulling a band of the interspinous stabilizer, and hold the metal hook to pass through the interspinous ligaments of the target spinous processes; (iii) guiding a metal hook to pass through the circular structure, and pull the band to form a knot on the circular structure; (iv) hold the metal hook to pass through a fixation ring, and slide the fixation ring toward the knot, and clamp the fixation ring on the band by a surgical tool to position the knot and prevent the knot from loosen. The interspinous stabilizer in accordance with the present disclosure is easier to be inserted between the vertebrae due to its asymmetrical double-wing structure. The dislocation of the interspinous stabilizer would be difficult after implantation, and the comfort of the patient after being implanted with the interspinous stabilizer is improved.

The present disclosure is directed to an interspinous stabilizer. The interspinous stabilizer comprises an interspinous spacer, and the interspinous spacer comprising a central support, and the central support having an upper side wing and an upper protrusion extending from the central support, and a lower side wing and a lower protrusion also extending from the central support. The upper side wing is longer than the upper protrusion and the lower side wing is longer than the lower protrusion to form an asymmetrical double-wing structure. The asymmetrical double-wing structure allows the surgeon to implant the interspinous spacer between 2 vertebrae of the patient from the lateral side during an operation. "Surgeon" refers to the person performing an operation to implant the interspinous stabilizer. "Patient" refers to the person suffering from spinal diseases and is in need of being surgically implanted with the interspinous stabilizer.

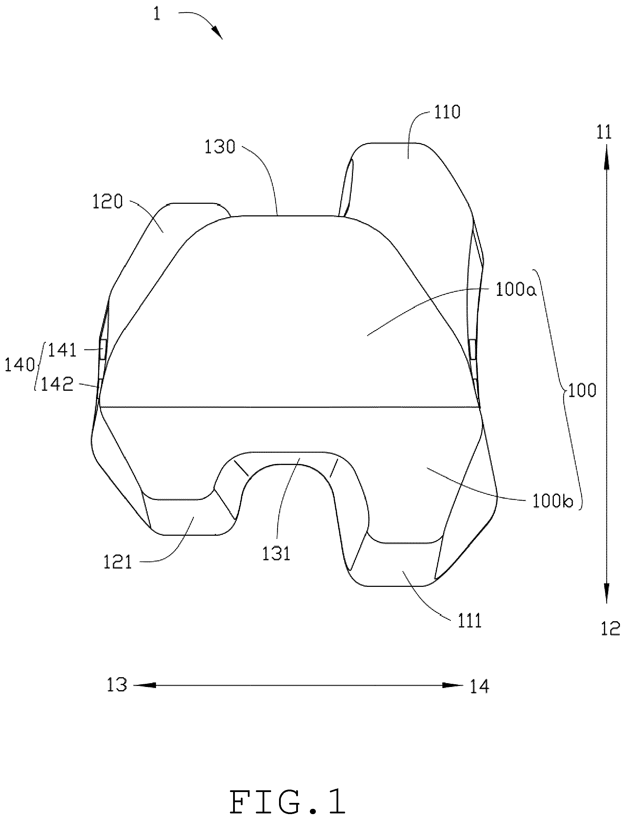

FIG. 1 illustrates an embodiment of the present disclosure, and it is an appearance of an interspinous spacer 1. The interspinous spacer 1 comprises a central support 100. The central support 100 having a first direction 13, a second direction 14, an upper side 11 and a lower side 12, and the first direction 13 and the second direction 14 are opposite to each other. The upper side 11 is perpendicular to both of the first direction 13 and the second direction 14. The lower side 12 is perpendicular to both of the first direction 13 and the second direction 14. A length of the central support 100 from the first direction 13 to the second direction 14 is about 15 mm to 25 mm. The central support may further comprise an upper support 100a on the upper side 11 and a lower support 100b on the lower side 12. A length of the upper support 100a from the upper side 11 to the lower side 12 is about 8 mm to 16 mm; a width of the upper support 100a from the first direction 13 to the second direction 14 is about 10 mm to 20 mm; a length of the lower support 100b from the upper side 11 to the lower side 12 is about 6 mm to 12 mm; a width of the lower support 100b from the first direction 13 to the second direction 14 is about 10 mm to 20 mm. An upper side wing 110 is on the second direction 14 and extending from the upper support 100a to the upper side 11, and a length of the upper side wing 110 is about 5.5 mm to about 15 mm. A lower side wing 111 is on the second direction 14 and extending from the lower support 100b to the lower side 12, and a length of the lower side wing 111 is about 5.5 mm to about 15 mm. An upper protrusion 120 is on the first direction 13 and extending from the upper support 100a to the upper side 11, and the upper protrusion 120 has a length of about 2 mm to 5 mm. A lower protrusion 121 is on the first direction 13 and extending from the lower support 100b to the lower side 12, and the lower protrusion has a length of about 2 mm to 5 mm. An upper concave surface 130, the upper concave surface 130 is between the upper side wing 110 and the upper protrusion 120 of the upper support 100a and facing toward the upper side 11, wherein the distance of the upper concave surface 130 from the first direction 13 to the second direction 14 is about 2 mm to about 6 mm. A lower concave surface 131, the lower concave surface 131 is between the lower side wing 111 and the lower protrusion 121 of the lower support 100b and facing toward the lower side 12, wherein the distance of the lower concave surface 130 from the first direction 13 to the second direction 14 is about 2 mm to about 6 mm. A set of perforations 140, comprising an upper perforation 141 and a lower perforation 142. The distance from the upper perforation 141 to a top end of the upper side 11 of the central support 100 is about 4 mm to about 6 mm. The distance from the lower perforation 142 to the top end of the upper side 11 of the central support 100 is about 7 mm to about 10 mm.

The upper protrusion 120 of the interspinous spacer 1 extends from the upper support 100a toward the upper side 11. The length of the upper protrusion 120 is shorter than the upper side wing 110. The upper side wing 110 extends from upper support 100a toward the upper side 11. The lower protrusion 121 extends from the lower support 100b toward the lower side 12. The length of the lower protrusion is shorter than the lower side wing 111. The lower side wing 111 extends from lower support 100b toward the lower side 12. Thus, the upper side wing 110 has a length larger than the upper protrusion 120 and the lower side wing 111 has a length larger than the lower protrusion 121 to form an asymmetrical double-wing structure having a longer upper side 11-lower side 12 axial extension on the second direction 14 than the first direction 13. The asymmetrical double-wing structure allows the surgeon to insert the interspinous spacer 1 between the two vertebrae of the patient from the lateral side of the spine during an operation. The asymmetrical double-wing structure fits the spine structure of the patient and increases the contact area with the spinous process, allowing an even distribution of stress to different parts of the two vertebrae. When spine rotation occurs, the interspinous spacer 1 would still be staying in the implantation location in the patient's spine. The durability of the interspinous spacer 1 inside the patient's body is improved.

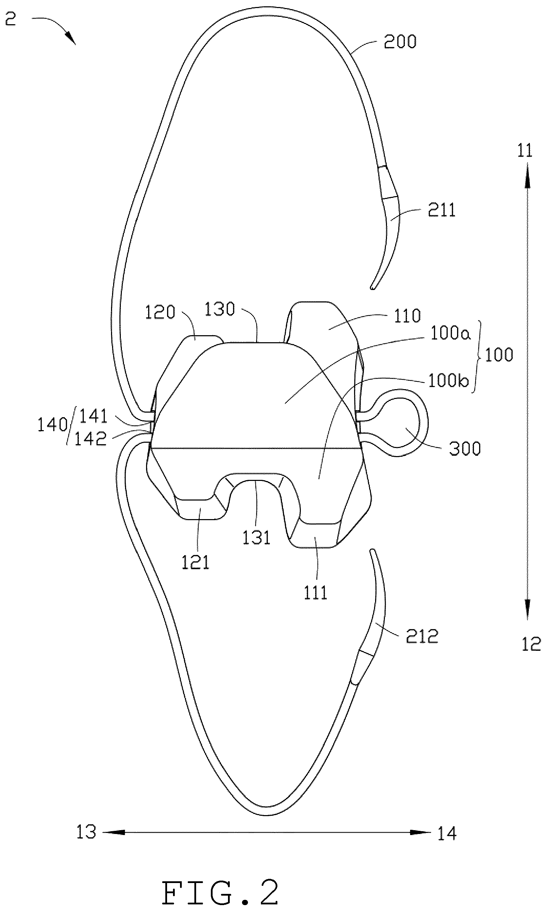

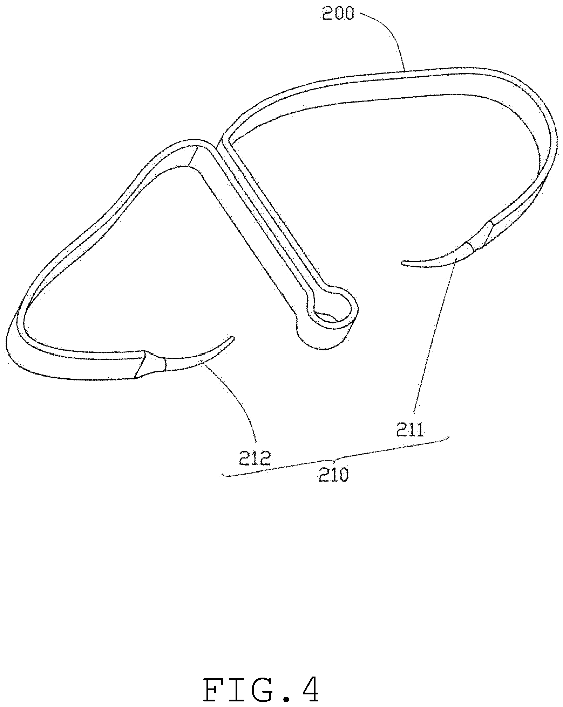

Referring to FIG. 2 and FIG. 4, appearances of an interspinous stabilizer 2 in accordance with the present disclosure are illustrated. The interspinous stabilizer 2 further comprises a band 200. The band 200 passes through one perforation of the perforation set 140 and forms a circular structure 300 at the second direction 14 of the central support 100. The band 200 having a flat or a round cross-section. A length of the band 200 is about 50 mm to about 300 mm, and the area of the cross-section of the band 200 is about 1 mm² to about 5 mm². When the interspinous spacer 1 is inserted between the vertebrae of the patient, a first metal hook 211 and a second metal hook 212 of the interspinous stabilizer 2 are directed by the surgeon to pass through the interspinous ligaments between the upper vertebra and the lower vertebra, and then pass through the circular structure 300. The surgeon then pulls the band 200 to reduce the size of the circular structure 300. The first metal hook 211 and the second metal hook 212 are fixed, and the interspinous stabilizer 2 is positioned on the implantation location between the vertebrae of the patient. The different parts of the spine contact with the band 200, therefore the different parts of the spine bear similar levels of stress. If more than one element is used to fix the interspinous stabilizer 2, then different parts of the spine may bear different levels of stress, and may cause spinal injury in the long term. The life quality of the patient can be affected by the spinal injury.

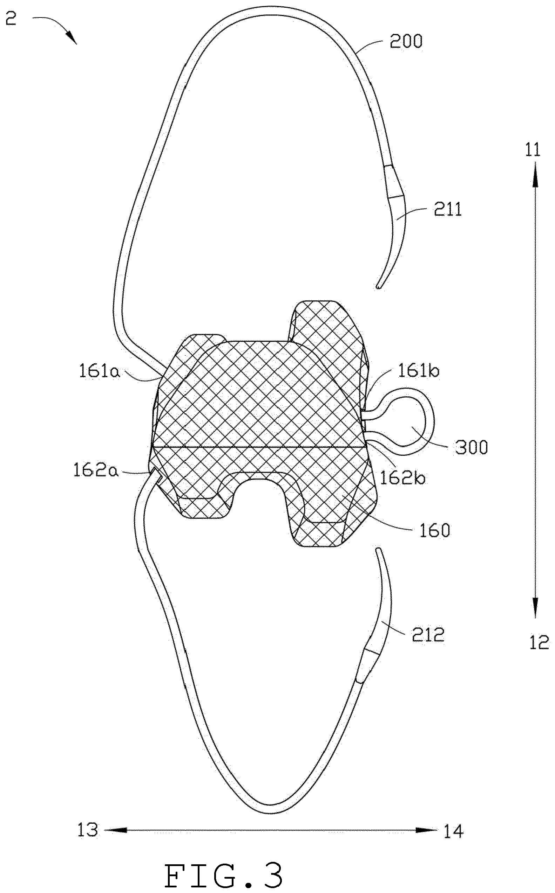

Referring to FIG. 3, an appearance of the interspinous stabilizer 2 and its' fabric sheath 160 in accordance with the present disclosure is illustrated. The interspinous stabilizer 2 is wrapped by a fabric sheath 160. The fabric sheath 160 could wrap the interspinous spacer 1 completely. The fabric sheath 160 having an upper first direction opening 161a, an upper second direction opening 161b, a lower first direction opening 162a and a lower second direction opening 162b for the trespassing of the band 200. The upper first direction opening 161a is closer to the upper side 11 than the upper perforation 141 in FIG. 1 and FIG. 2. The lower first direction opening 162a is closer to lower side 12 than the lower perforation 142 in FIG. 1 and FIG.2. When the band 200 leaves from the interspinous spacer 1, it respectively folds toward the upper side 11 and the lower side 12 in the space between the fabric sheath 160 and the interspinous spacer 1, in order to pass through the upper first direction opening 161a and the lower first direction 162a. The fabric sheath 160 could serve as a cushion between the interspinous stabilizer 2 and the spine. The fabric sheath 160 prevents the interspinous spacer 1 from make direct contact with the vertebrae, causing abrasion to the interspinous spacer 1. The fabric sheath 160 also effectively reduces the tissue damage around the spinous processes from the interspinous stabilizer 2.

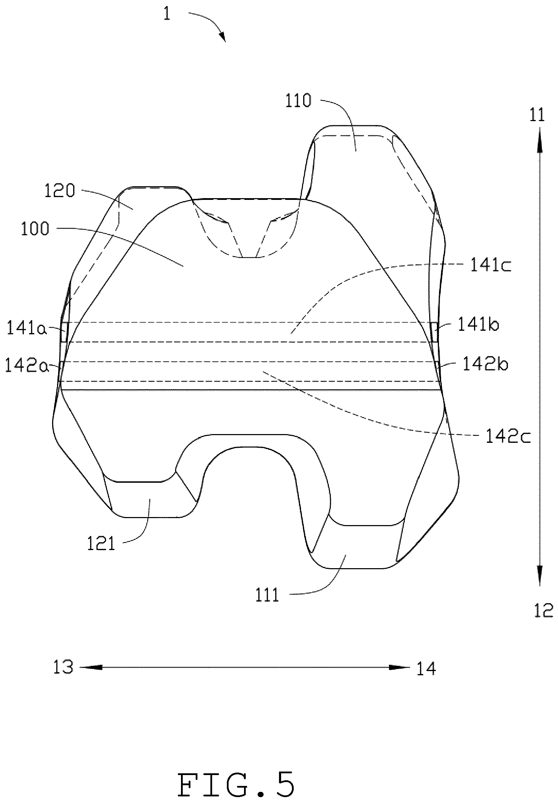

Referring to FIG. 5, a perspective view of the interspinous spacer 1 in accordance with the present disclosure is illustrated. One end of the band 200 is introduced into the upper second direction perforation 141b and passes through a conduit for the upper perforation 141c, then leaves via the upper first direction perforation 141a. The other end of the band 200 enters the lower second direction perforation 142b and passes through a conduit for the lower perforation 142c, then leaves via the lower first direction perforation 142a. The conduit for the upper perforation 141c and the conduit for the lower perforation 142c are inside the central support 100, and the conduit for the upper perforation 141c and the conduit for the lower perforation 142c do not cross inside the central support 100. Preferably, the conduit for the upper perforation 141c and the conduit for the lower perforation 142c are two parallel passages.

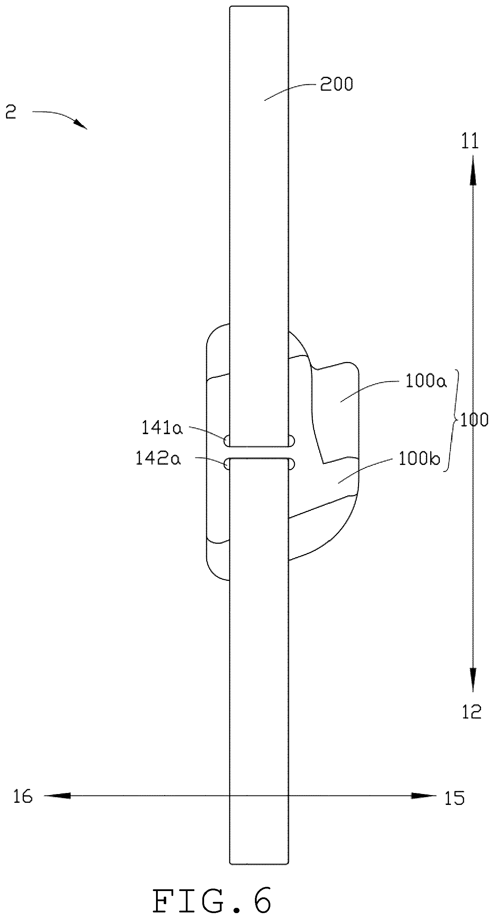

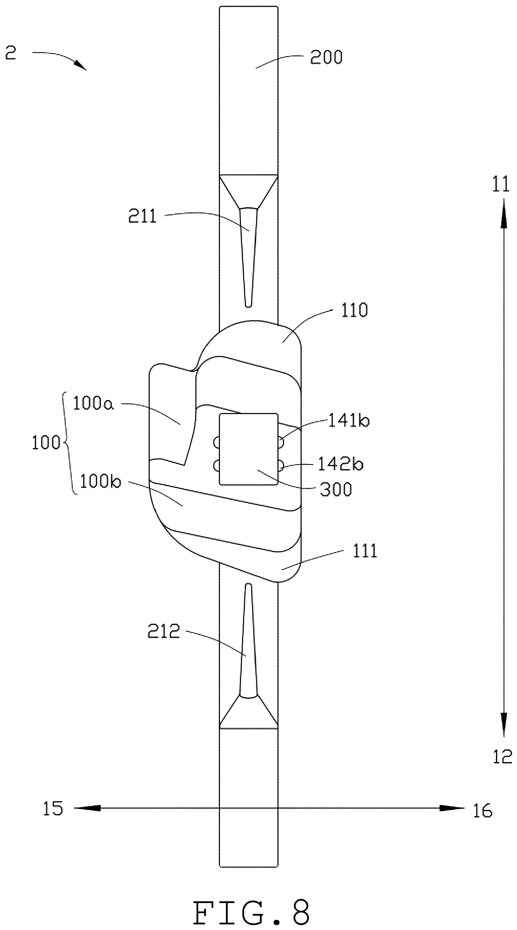

Referring to FIG. 6 and FIG. 8, side views of the interspinous stabilizer 2 from the first direction 13 and the second direction 14 in accordance with the present disclosure are illustrated. A front side 15 and a rear side 16 is defined in the present disclosure: the front side 15 and the rear side 16 is opposite to each other, and the front side 15 and the rear side 16 are perpendicular to both of the first direction 13 and the second direction 14, and the front side 15 and the rear side 15 are perpendicular to both of the upper side 11 and the lower side 12. After the implantation of the interspinous spacer 1 or the interspinous stabilizer 2 between the vertebrae of the patient's spine, the front side 15 will face the spinal column of the patient, and the rear side 16 will face the dorsal skin of the patient.

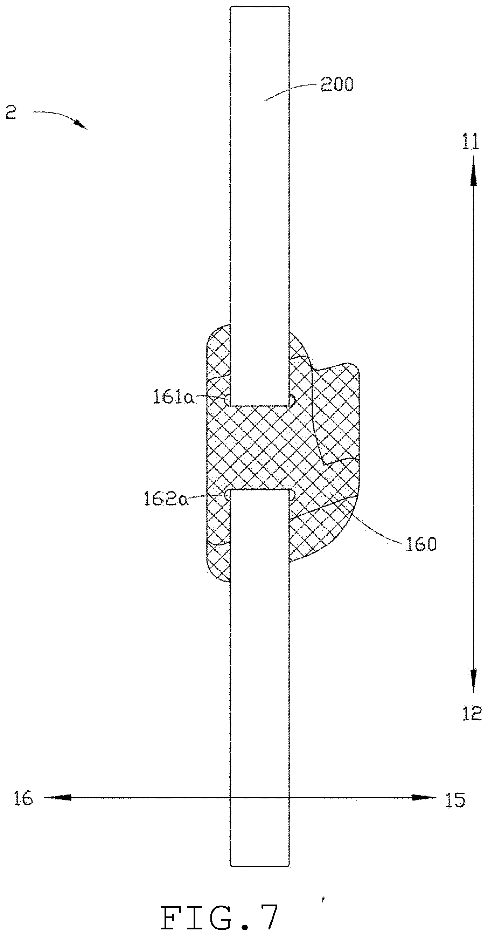

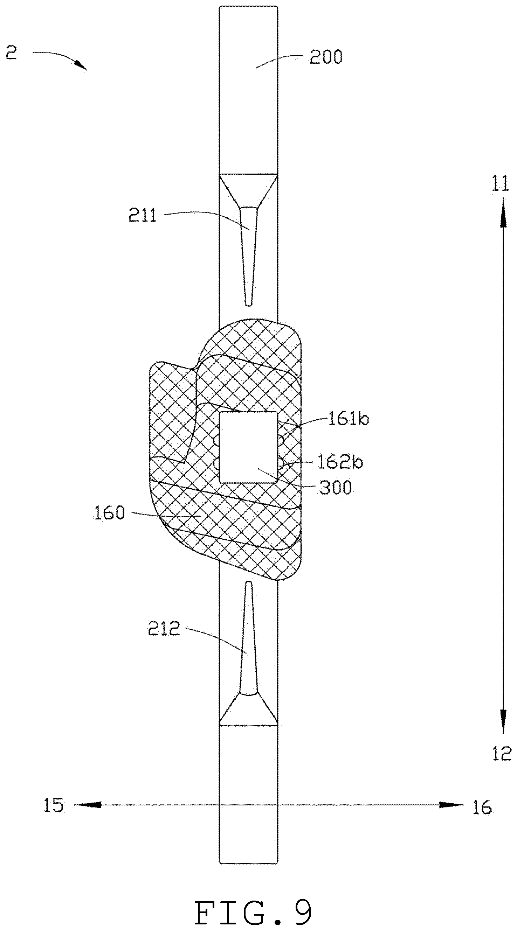

Referring to FIG. 7 and FIG. 9, side views of the interspinous stabilizer 2 and the fabric sheath 160 from the first direction 13 and the second direction 14 in accordance with the present disclosure are illustrated. It can be understood from FIG. 8 and FIG. 9 that the band 200 leave the interspinous spacer 1 from the upper second direction perforation 141b, and leaves the fabric sheath 160 from the second upper direction opening 161b. On the lower side 12 and the second direction 14, the band 200 leaves the interspinous spacer 1 from the lower second direction perforation 142b, and leaves the fabric sheath 160 from the lower second direction opening 162b. It can be understood from FIG. 6 and FIG. 7 that the band 200 leaves the interspinous spacer 1 from the upper first direction perforation 141a, and folds upwardly to leave the fabric sheath 160 from the upper first direction opening 161a. When the interspinous spacer 1 is wrapped with the fabric sheath 160, the position of the upper first direction opening 161a is not overlapped with the upper first direction perforation 141a. On the lower side 12 and the first direction 13, the band 200 leaves the interspinous spacer 1 from the lower first direction perforation 142a, and folds downwardly to leave the fabric sheath 160 from the lower first direction opening 162a. When the interspinous spacer 1 is wrapped with the fabric sheath 160, the position of the lower first direction opening 162a is not overlapped with the lower first direction perforation 142a.

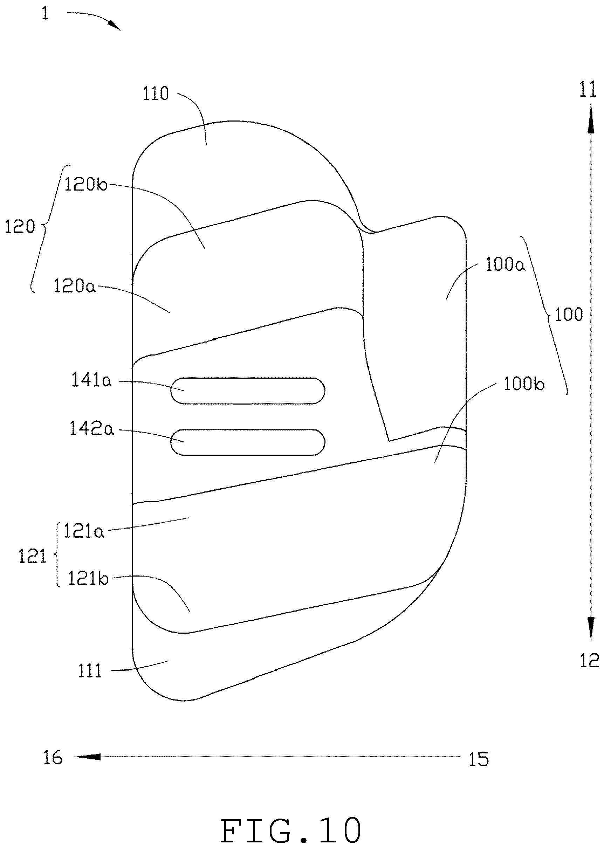

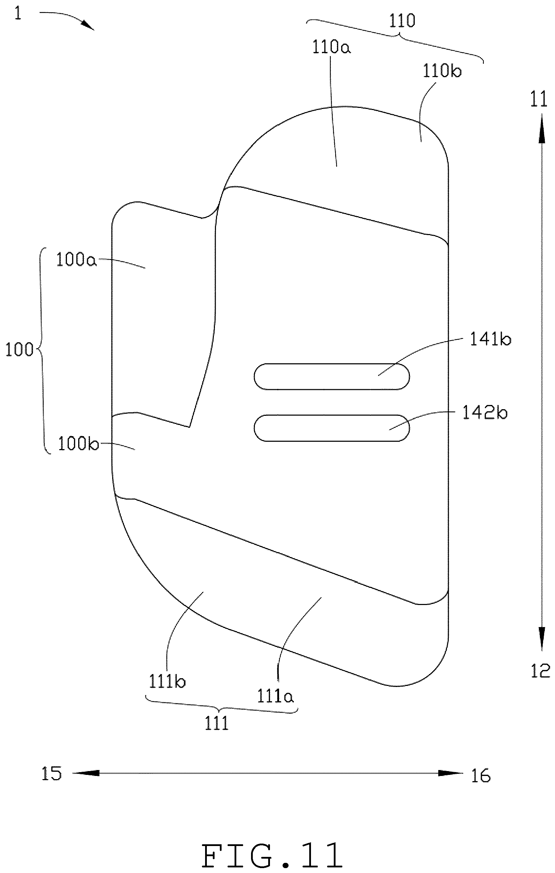

Referring to FIG. 10 and FIG. 11, side views of the interspinous spacer 1 from the first direction 13 and the second direction 14 in accordance with the present disclosure are illustrated. Each of the upper side wing 110, the lower side wing 111, the upper protrusion 120 and the lower protrusion 121 includes a root portion that is closer to the central support 100 and an end portion that is further to the central support 100, denoted by a root portion of the upper side wing 110a, an end portion of the upper side wing 110b, a root portion of the lower side wing 111a, an end portion of the lower side wing 111b, a root portion of the upper protrusion 120a, an end portion of the upper protrusion 120b, a root portion of the lower protrusion 121a and an end portion of the lower protrusion 121b. "Thickness" refers to the space occupied by one or all parts of the interspinous spacer 1 in an axis formed from the front side 15 to the rear side 16. A thickness of the upper side wing 110 is smaller than the lower side wing 111 of the interspinous spacer 1, wherein a thickness of the upper side wing 110 is about 6 mm to about 10 mm and a thickness of the lower side wing is about 10.5 mm to about 15 mm. A thickness of the upper protrusion 120 is smaller than the lower protrusion 121 of the interspinous spacer 1, wherein a thickness of the upper protrusion 120 is about 6 mm to about 10 mm and a thickness of the lower protrusion 121 is about 10.5 mm to about 15 mm. The thickness of the upper support 100a and the lower support 100b of the interspinous spacer 1 are about 8 mm to about 20 mm. The thickness of the upper side wing 110 is smaller than the thickness of the upper support 100a of the interspinous spacer 1. The different thickness of above structures of the interspinous spacer 1 has a better fitting with the human spine structure, thus the upper concave surface 130 between the upper side wing 110 and the upper protrusion 120 would receive the spinous process of the patient. After the interspinous spacer 1 is implanted between the vertebrae of the spine, the different thickness of above structures ensures that the part receiving most stress is located beneath the vertebral plate, to have a better positioning of the interspinous spacer 1. The different thickness of above structures also allows the interspinous stabilizer 2 to have an implantation location that is closer to the rotation center of the spinal column. Each of the root portion 110a, 111a, 120a and 121a of the above parts is connected to the end portion 110b, 111b, 120b and 121b to form a transitional edge. On the first direction 13, the transitional edges make the band 200 closer to the spinous processes when the band 200 is fixing the interspinous stabilizer 200, thus provides a better positioning. On the second direction 14, the transitional edges allow an easier implantation, wherein the surgeon laterally inserts the interspinous spacer 1 between 2 vertebrae of the patient.

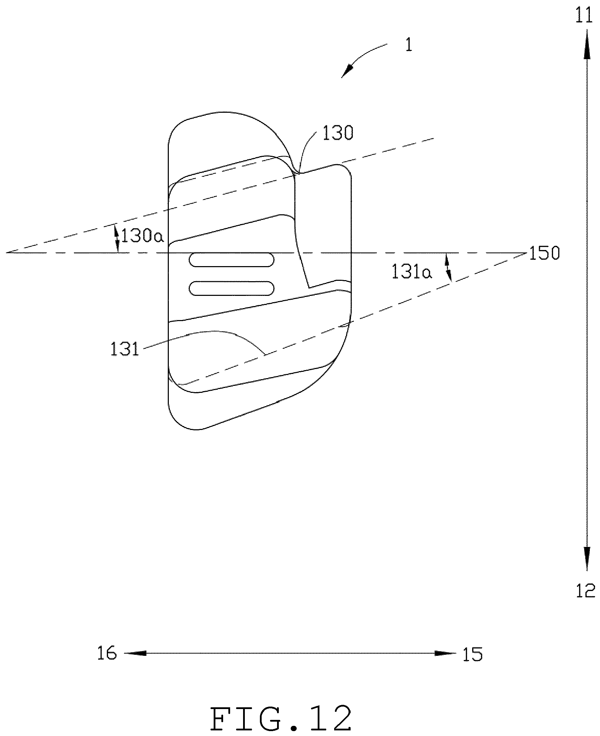

Referring to FIG. 12, it is a cross-sectional view of the interspinous spacer 1 on the first direction 13. A horizontal plane 150 is formed from the front side 15 stretching to the rear side 16. The horizontal plane 150 is perpendicular to both the upper side 11 and the lower side 12. The upper concave surface 130 is inclined to the rear side 16 from the front side 15, thus forms an upper angle 130a. The upper angle 130a is ranged from 0• to 60•, preferably, the upper angle 130a should be between 10• to 45• The lower concave surface 131 forms a lower angle 131a with the horizontal plane 150, wherein the lower angle 131a is ranged from 0• to 60•, preferably, the lower angle 131a should be 10• to 45• "Inclination" of "incline" describes a particular surface of part of the interspinous spacer 1 that is not parallel to the horizontal plane 150. The particular surface or part may be tilted to the upper side 11 or the lower side 12 along the front side 15 to the rear side 16. Because the upper angle 130a does not equal to the lower angle 131a, therefore the upper concave surface 130 is not parallel with the lower concave surface 131, and this structural feature provides an improved fitting between the interspinous spacer 1 and the spinal structure of the patient.

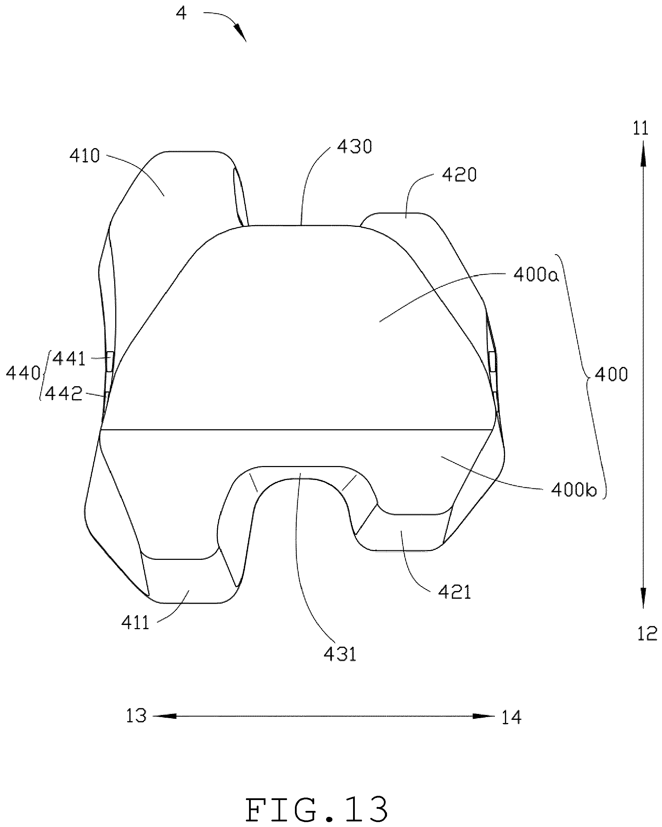

FIG. 13 is another embodiment of the present disclosure, and it illustrate an appearance of an interspinous spacer 4. The interspinous spacer 4 comprises a central support 400. The central support 400 may further comprise an upper support 400a on the upper side 11 and a lower support 400b on the lower side 12. An upper side wing 410 is on the first direction 13 and extending from the upper support 400a to the upper side 11. A lower side wing 411 is on the first direction 13 and extending from the lower support 400b to the lower side 12. An upper protrusion 420 is on the second direction 14 and extending from the upper support 400a to the upper side 11. A lower protrusion 421 is on the second direction 14 and extending from the lower support 400b to the lower side 12. An upper concave surface 430, the upper concave surface 430 is between the upper side wing 410 and the upper protrusion 420 of the upper support 400a and facing toward the upper side 11. A lower concave surface 431, the lower concave surface 431 is between the lower side wing 411 and the lower protrusion 421 of the lower support 400b and facing toward the lower side 12. A set of perforations 440, comprising an upper perforation 441 and a lower perforation 442.

The upper protrusion 420 of the interspinous spacer 4 extends from the upper support 400a toward the upper side 11. A length of the upper protrusion 420 is shorter than the upper side wing 410. The lower protrusion 421 extends from the lower support 400b toward the lower side 12. The length of the lower protrusion 421 is shorter than the lower side wing 411. Thus, the upper side wing 410 has a length larger than the upper protrusion 420 and the lower side wing 411 has a length larger than the lower protrusion 421 to form an asymmetrical double-wing structure having a longer extension on the first direction 13 than the second direction 14.

Referring to FIG. 13, it illustrates an appearance of another interspinous spacer 4 in accordance with the present disclosure. A length of the central support 400 from the first direction 13 to the second direction 14 is about 15 mm to 25 mm. The central support may further comprise an upper support 400a on the upper side 11 and a lower support 400b on the lower side 12. A length of the upper support 400a from the upper side 11 to the lower side 12 is about 8 mm to 16 mm; a width of the upper support 400a from the first direction 13 to the second direction 14 is about 10 mm to 20 mm; a length of the lower support 400b from the upper side 11 to the lower side 12 is about 6 mm to 12 mm; a width of the lower support 400b from the first direction 13 to the second direction 14 is about 10 mm to 20 mm. An upper side wing 410 is on the first direction 13 and extending from the upper support 400a to the upper side 11, and a length of the upper side wing 410 is about 5.5 mm to about 15 mm. A lower side wing 411 is on the first direction 13 and extending from the lower support 400b to the lower side 12, and a length of the lower side wing 411 is about 5.5 mm to about 15 mm. An upper protrusion 420 is on the second direction 14 and extending from the upper support 400a to the upper side 11, and the upper protrusion 420 has a length of about 2 mm to 5 mm. A lower protrusion 421 is on the second direction 14 and extending from the lower support 400b to the lower side 12, and the lower protrusion 421 has a length of about 2 mm to 5 mm. An upper concave surface 430, the upper concave surface 430 is between the upper side wing 410 and the upper protrusion 420 of the upper support 400a and facing toward the upper side 11, wherein the distance of the upper concave surface 430 from the first direction 13 to the second direction 14 is about 2 mm to about 6 mm. A lower concave surface 431, the lower concave surface 431 is between the lower side wing 411 and the lower protrusion 421 of the lower support 400b and facing toward the lower side 12, wherein the distance of the lower concave surface 430 from the first direction 13 to the second direction 14 is about 2 mm to about 6 mm. A set of perforations 440, comprising an upper perforation 441 and a lower perforation 442. The distance from the upper perforation 441 to a top end of the upper side 11 of the central support 400 is about 4 mm to about 6 mm. The distance from the lower perforation 442 to the top end of the upper side 11 of the central support 400 is about 7 mm to about 10 mm.

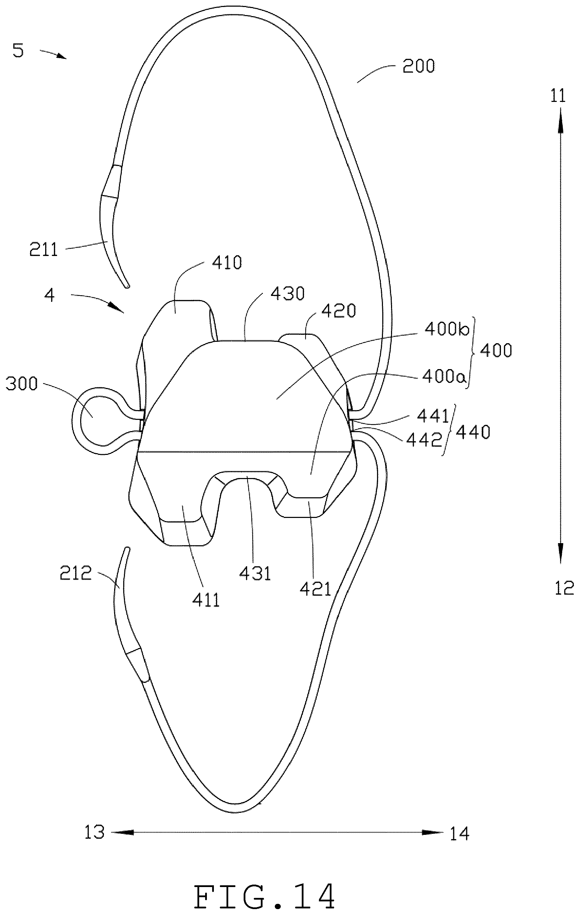

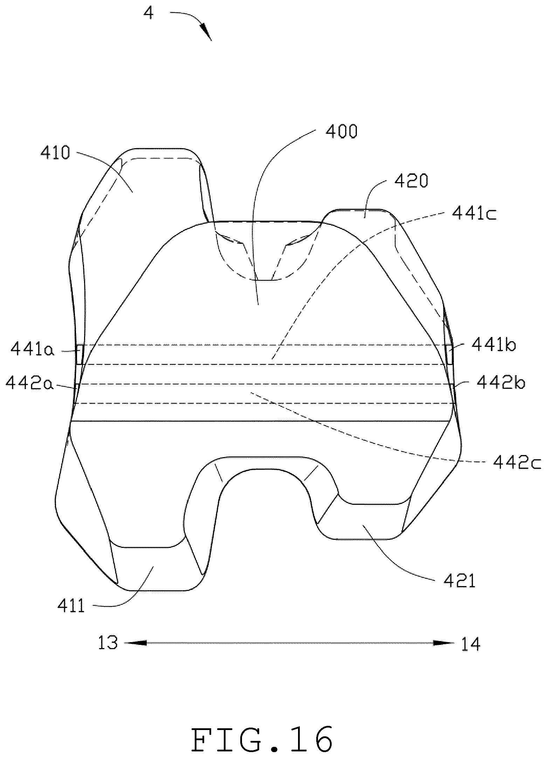

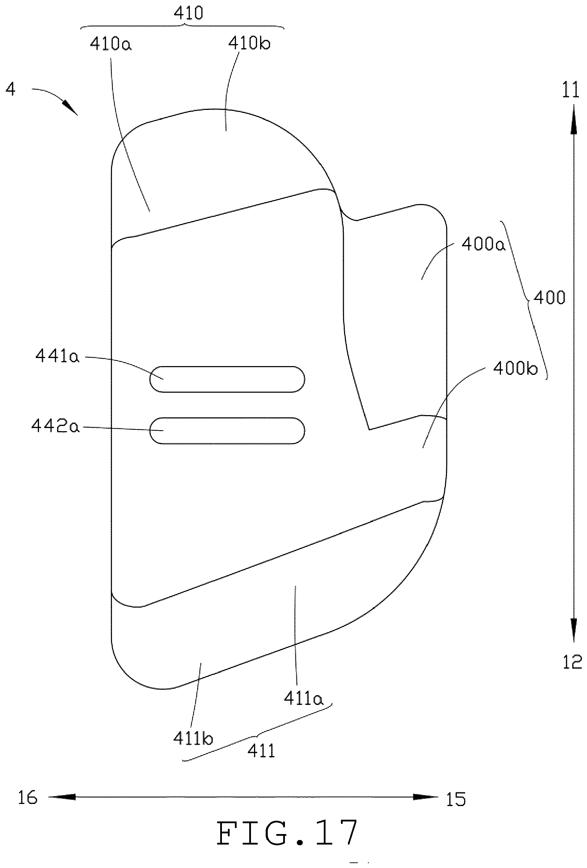

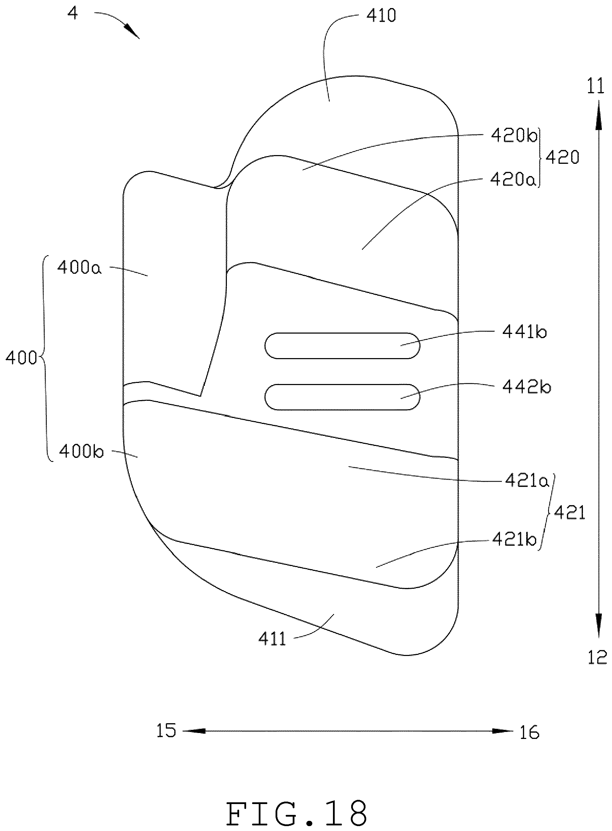

FIG. 14 is an appearance of an interspinous stabilizer 5, FIG. 16 is a perspective view of an interspinous spacer 4, FIG. 17 is a side view of the interspinous spacer 4 from the first direction 13 and FIG. 18 is a side view of the interspinous spacer 4 from the second direction 14, in accordance with the present disclosure. The interspinous stabilizer 5 further comprises a band 200. The band 200 passes through one perforation in the perforation set 440 and forms a circular structure 300 at the first direction 13 of the central support 400. The interspinous stabilizer 5 further comprises a set of metal hooks 210, including a first metal hook 211 and a second metal hook 212. When the interspinous spacer 4 is implanted between the vertebrae of the patient, a first metal hook 211 of the interspinous stabilizer 5 are directed by the surgeon to pass through the interspinous ligaments between the upper vertebra and the lower vertebra, and then pass through the circular structure 300. The surgeon then pulls the band 200 to reduce the size of the circular structure 300. The first metal hook 211 and the second metal hook 212 are fixed, and the interspinous stabilizer 5 is positioned in the implantation location between the vertebrae of the patient. The different parts of the spine contact with the band 200, therefore the different parts of the spine bear similar levels of stress. After the implantation of the interspinous spacer 4 or the interspinous stabilizer 5 between the vertebrae of the patient's spine, the front side 15 will face the spinal column of the patient, and the rear side 16 will face the dorsal skin of the patient.

Each of the upper side wing 410, the lower side wing 411, the upper protrusion 420 and the lower protrusion 421 includes a root portion that is closer to the central support 400 and an end portion that is further to the central support 400, denoted by a root portion of the upper side wing 410a, an end portion of the upper side wing 410b, a root portion of the lower side wing 411a, an end portion of the lower side wing 411b, a root portion of the upper protrusion 420a, an end portion of the upper protrusion 420b, a root portion of the lower protrusion 421a and an end portion of the lower protrusion 421b. The thickness of the upper side wing 410 is smaller than the lower side wing 411 of the interspinous spacer 4. The thickness of the upper protrusion 420 is smaller than the lower protrusion 421 of the interspinous spacer 4. The different thickness of above structures of the interspinous 4 allows the interspinous stabilizer 5 to have an implantation location that is closer to the rotation center of the spinal column. Each of the root portion 410a, 411a, 420a and 421a is connected to the end portion 410b, 411b, 420b and 421b of the above structures to form a transitional edge. The transitional edges allow the band 200 to be closer to the spinous processes of the patient when fixing the interspinous stabilizer 5, in order to provide a better positioning. The transitional edges also allow an easier implantation, wherein the surgeon laterally inserts the interspinous spacer 4 between two vertebrae of the patient. The thickness of the upper side wing 410 is smaller than the thickness of the upper support 400a of the interspinous spacer 4. The thickness of the upper protrusion 420 is smaller than the thickness of the upper support 400a. The different thickness of above structures of the interspinous spacer 4 has a better fitting with the human spine structure, thus an upper concave surface 430 between the upper side wing 410 and the upper protrusion 420 would receive the spinous process of the patient. After the interspinous spacer 4 is implanted between the vertebrae of the spine, the different thickness of above structures ensures that the part receiving most stress is located beneath the vertebral plate, to have a better positioning of the interspinous spacer 4.

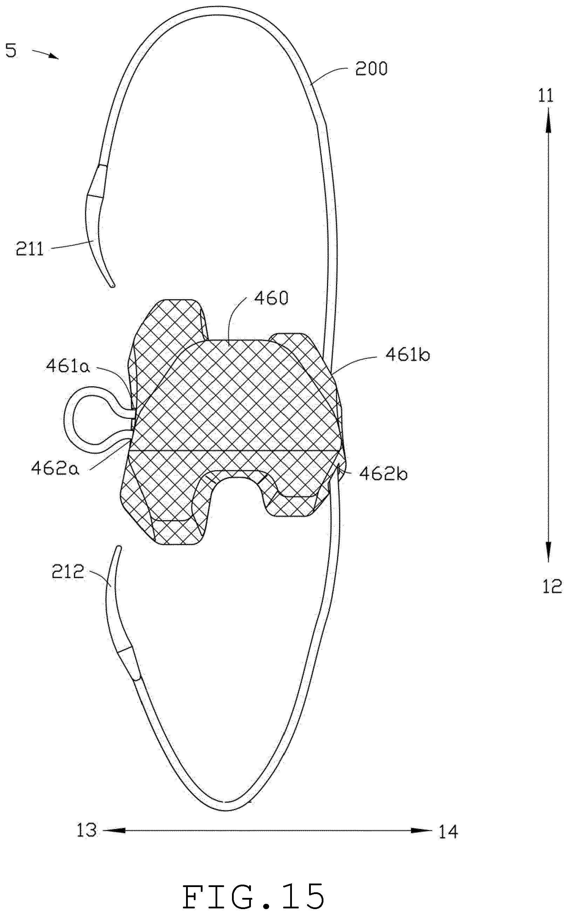

Referring to FIG. 15, an appearance of the interspinous stabilizer 5 and a fabric sheath 460 in accordance with the present disclosure is illustrated. The interspinous stabilizer 5 is wrapped by a fabric sheath 460. The fabric sheath 460 could wrap the interspinous spacer 4 completely. The fabric sheath 460 having an upper first direction opening 461a, an upper second direction opening 461b, a lower first direction opening 462a and a lower second direction opening 462b for the trespassing of the band 200. The upper first direction opening 461a is closer to the upper side 11 than the upper perforation 441 in FIG. 16 and FIG. 17. The lower first direction opening 462a is closer to the lower side 12 than the lower perforation 442 in FIG. 16 and FIG.18. When the band 200 leaves from the interspinous spacer 4, it respectively folds toward the upper side 11 and the lower side 12 in the space between the fabric sheath 460 and the interspinous spacer 4, in order to pass through the upper first direction opening 461a and the lower first direction 462a.

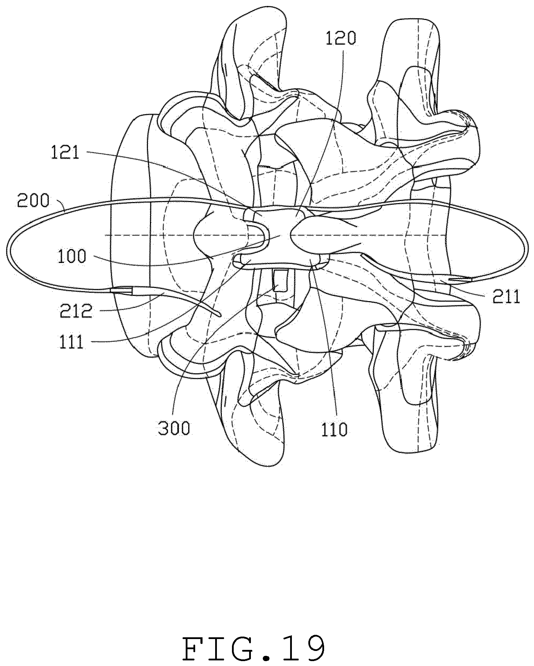

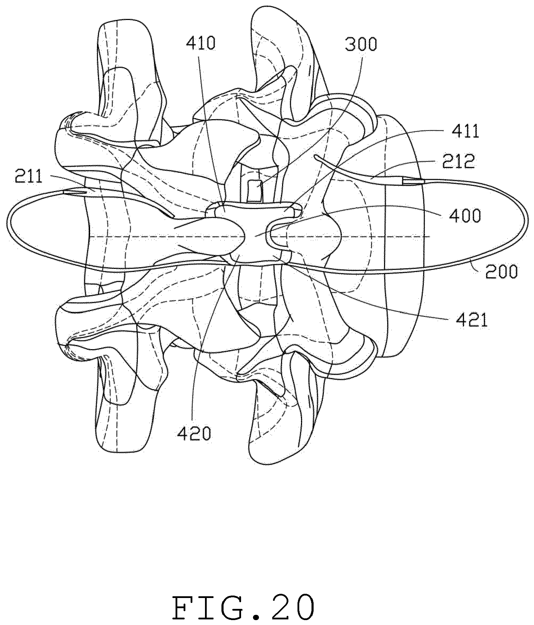

Referring to FIG. 19 and FIG. 20, top views of the interspinous stabilizer 2 and the interspinous stabilizer 5 in accordance with the present disclosure are illustrated. The present disclosure provides a method of stabilizing relative position between the human spinous processes, comprising: (i) insert the interspinous stabilizer 2 or the interspinous stabilizer 5 between two spinous processes of the patient; (ii) pull the band 200 of the interspinous stabilizer 2 or the interspinous stabilizer 5, and hold the metal hook 210 to pass through the interspinous ligaments of target spinous processes; (iii) hold the metal hook 210 to pass through the circular structure 300, and pull the band 200 to form a knot on the circular structure 300; (iv) hold the metal hook 210 to pass through a fixation ring, and slide the fixation ring toward the knot, and clamp the fixation ring on the band 200 by a surgical tool to position the knot and prevent the knot from loosen. The target spinous processes in the above method can be spinous processes of any two of the lumbar vertebrae.

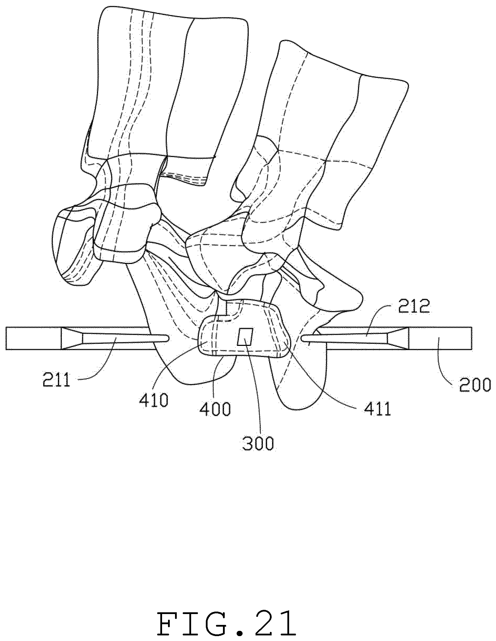

Referring to FIG. 21, a side view on the first direction 13 of the interspinous stabilizer 5 in accordance with the present disclosure being implanted between the vertebrae of the patient is illustrated. The metal hook 210 is capable to pass through the spinous processes between two lumbar vertebrae, and then pass through the circular structure 300 to fix the interspinous stabilizer 5.

The forth mentioned method for implanting the interspinous stabilizer 2 between two spinous processes of the patient can be: inserts the interspinous stabilizer 2 from the first direction 13 to the space between the spinous processes of the patient; adjusts the interspinous stabilizer 2, and the lower concave surface 131 should first be aligned with and receive a lower spinous process of the patient, then the upper concave surface 130 should be aligned with and receive an upper spinous process of the patient; and fixes the interspinous stabilizer 2 onto its' implantation location by the circular structure 300 and a fixation ring. The forth mention method for implanting the interspinous stabilizer 5 between two spinous processes of the patient can be: inserts the interspinous stabilizer 5 from the second direction 14 to the space between the spinous processes of the patient; adjusts the interspinous stabilizer 5, and the lower concave surface 431 should first be aligned with and receive a lower spinous process of the patient, then the upper concave surface 430 should be aligned with an receive an upper spinous process of the patient; and fixes the interspinous 5 onto its' implantation location by the circular structure 300 and a fixation ring. The fabric sheath 160 for the interspinous stabilizer 2, the fabric sheath 460 for the interspinous stabilizer 5 and the band 200 can be assembled before the operation.

The interspinous spacer 1 and the interspinous spacer 4 can be comprised of dimethyl silicone or polyurethane, or the combination thereof. Preferably, dimethyl silicone could be the core of the interspinous spacer 1 or the interspinous spacer 4, and the core could be covered by polyurethane. The band 200 can be comprised of polyester fiber or highly cross-linked polyethylene fiver. The metal hook 210 can be comprised of titanium alloy, stainless steel or any other biocompatible metal materials. The fabric sheath 160 and the fabric sheath 460 can be comprised of polyester fiber or highly cross-linked polyethylene fiber. The fixation ring can be comprised of titanium alloy, medical grade stainless steel or any other biocompatible metal materials.

The foregoing descriptions of specific compositions and methods of the present disclosure have been presented for purposes of illustration and description. They are not intended to be exhaustive or to limit the disclosure to the precise compositions and methods disclosed and obviously many modifications and variations are possible in light of the above teaching. The examples were chosen and described in order to best explain the principles of the disclosure and its practical application, to thereby enable others skilled in the art to best utilize the disclosure with various modifications as are suited to the particular use contemplated. It is intended that the scope of the disclosure be defined by the claims appended hereto and their equivalents.

## Claims

1. An interspinous spacer, comprising:
a central support comprising a first direction, a second direction opposite to the first direction, an upper side perpendicular to the first direction and the second direction, a lower side perpendicular to the first direction and the second direction;
an upper side wing extending from the upper side of the central support and on one of the first direction and the second direction;
a lower side wing extending from the lower side of the central support on a direction identical to the upper side wing, wherein a thickness of the upper side wing is smaller than a thickness of the lower side wing;
an upper protrusion extending from the upper side of the central support and on another of the first direction and the second direction, wherein a length of the upper protrusion is shorter than a length of the upper side wing;
a lower protrusion extending from the lower side of the central support and on a direction identical to the upper protrusion, wherein a length of the lower protrusion is shorter than a length of the lower side wing, and a thickness of the upper protrusion is smaller than a thickness of the lower protrusion; and
at least one perforation traversing the central support from the first direction to the second direction;
wherein an upper concave surface of the central support is between the upper side wing and the upper protrusion and facing the upper side, a lower concave surface is between the lower side wing and the lower protrusion and facing the lower side, and the upper concave surface is not parallel with the lower concave surface.

2. The interspinous spacer of claim 1, wherein the central support further comprises an upper support and a lower support, the upper support is on the upper side of the central support and is connected to the upper protrusion, and the lower support is on the lower side of the central support and is connected to the lower protrusion.

3. The interspinous spacer of claim 2, wherein the thicknesses of the upper side wing and the upper protrusion are smaller than a thickness of the upper support, and a thicknesses of the lower side wing and the lower protrusion are smaller than a thickness of the lower support.

4. The interspinous spacer of claim 1, wherein the perforation comprises an upper perforation and a lower perforation, the upper perforation and the lower perforation traverse through the central support, and a distance between the upper perforation and a top of the central support is shorter than a distance between the lower perforation and the top of the central support.

5. The interspinous spacer of claim 1, wherein each of the upper side wing, the lower side wing, the upper protrusion and the lower protrusion comprises a root portion close to the central support and an end portion away from the central support,
thicknesses of the root portions of the upper side wing, the lower side wing, the upper protrusion and the lower protrusion are greater than thicknesses of the end portions of the upper side wing, the lower side wing, the upper protrusion and the lower protrusion.

6. The interspinous spacer of claim 1, wherein the central support further comprises a front side and a rear side, and the front side and the rear side are perpendicular to the first direction, the second direction, the upper side, and the lower side, and a horizontal plane extending from the front side to the rear side is perpendicular to the upper side and the lower side.

7. The interspinous spacer of claim 1, wherein a material of the interspinous spacer comprises dimethyl silicone, polyurethane or a combination thereof.

8. An interspinous stabilizer, comprising:
a interspinous spacer, comprising:
a central support comprising a first direction, a second direction opposite to the first direction, an upper side perpendicular to the first direction and the second direction, a lower side perpendicular to the first direction and the second direction;
an upper side wing extending from the upper side of the central support and on one of the first direction and the second direction;
a lower side wing extending from the lower side of the central support on a direction identical to the upper side wing, wherein a thickness of the upper side wing is smaller than a thickness of the lower side wing;
an upper protrusion extending from the upper side of the central support and on another of the first direction and the second direction, wherein a length of the upper protrusion is shorter than a length of the upper side wing;
a lower protrusion extending from the lower side of the central support and on a direction identical to the upper protrusion, wherein a length of the lower protrusion is shorter than a length of the lower side wing, and a thickness of the upper protrusion is smaller than a thickness of the lower protrusion; and
at least one perforation traversing the central support from the first direction to the second direction;
wherein an upper concave surface of the central support is between the upper side wing and the upper protrusion and facing the upper side, a lower concave surface is between the lower side wing and the lower protrusion and facing the lower side, and the upper concave surface is not parallel with the lower concave surface.
a band passing through the perforation and forming a circular structure on the first direction of the central support; and
at least one metal hook disposed on at least one end of the band and passing through the circular structure.

9. The interspinous stabilizer of claim 8, further comprising a fabric sheath covering at least a portion of the interspinous spacer and at least a portion of the band.

10. The interspinous stabilizer of claim 9, wherein the fabric sheath is comprised of polyester fabric or polyethylene fabric.

11. The interspinous stabilizer of claim 8, wherein the band is comprised of polyester fabric or polyethylene fabric.

12. The interspinous stabilizer of claim 8, wherein the metal hook is comprised of titanium alloy or stainless steel.

13. A method of stabilizing human spinous processes, comprising:
(i) inserting an interspinous stabilizer of claim 9 between two target spinous processes of a spine;
(ii) pulling a band of the interspinous stabilizer and holding at least one metal hook of the interspinous stabilizer to pass through interspinous ligaments of the target spinous processes;
(iii) guiding the metal hook to pass through a circular structure of the interspinous stabilizer and pulling the band to form a knot on the circular structure; and
(iv) holding the metal hook to pass through a fixation ring, sliding the fixation ring toward the knot, and clamp the fixation ring on the band by a surgical tool to position the knot.
